# EUROPEAN PATENT SPECIFICATION

(11) **EP 2 583 956 B1**
(45) Date of publication and mention of the grant of the patent: **08.01.2020**
(21) Application number: 12187708.8
(22) Date of filing: 08.10.2012
(51) Int. Cl.: C07D 333/56, C07D 407/04, C07D 409/04, C07D 491/14, C07D 209/80, B01J 19/10, C07D 333/76, C07D 409/14, C07D 405/14, C07D 317/70

(54) **A method of preparation of polycyclic, fused aromatic and heteroaromatic hydrocarbons and intermediates**
Méthode de préparation d'hydrocarbures polycycliques, aromatiques et hétéroaromatiques et des intermédiaires
Verfahren zur Herstellung polyzyklischer, kondensierter, aromatischer und heteroaromatischer Kohlenwasserstoffe und Zwischenprodukte

(30) Priority: 19.10.2011 PL 39670011
(43) Date of publication of application: 24.04.2013
(73) Proprietor: Centrum Badan Molekularnych I Makromolekularnych Pan, 90-363 Lodz (PL)
(72) Inventor: Balczewski,, Piotr, 91-225 Lódz (PL); Bodzioch,, Agnieszka, 28-366 Malogoszcz, (PL); Skalik, Joanna, 42-270 Klomnice, (PL)
(74) Representative: Wroblewski, Michal

(56) References cited:
- PL-A1- 385 794
- PL-A1- 389 778
- AGNIESZKA BODZIOCH ET AL: "Synthesis and Optoelectronic Properties of Hexahydroxylated 10-O-RSubstituted Anthracenes via a New Modification of the Friedel-Crafts Reaction Using O-Protected ortho-Acetal Diarylmethanols", CHEMISTRY- A EUROPEAN JOURNAL, vol. 18, 21 March 2012 (2012-03-21), pages 4866-4876, XP055049553, DOI: 10.1002/chem.201101909 -& A. Bodzioch ET AL: "Supporting Information - Synthesis and Optoelectronic Properties of Hexahydroxylated 10-O-RSubstituted Anthracenes via a New Modification of the Friedel-Crafts Reaction Using O-Protected ortho-Acetal Diarylmethanols", Chemistry - A European Journal, 21 March 2012 (2012-03-21), pages 1-20, XP055079276, Retrieved from the Internet: URL:http://onlinelibrary.wiley.com/store/1 0.1002/chem.201101909/asset/supinfo/chem_2 01101909_sm_miscellaneous_information.pdf? v=1&s=08918cf5ce9e331ef05acb5dc740b3b3a141 7fcb [retrieved on 2013-09-
- PIOTR BALCZEWSKI ET AL: "Unusual Transformation of the Diarylmethanol Derivative into an Unknown 1,2,3,6,7,10-Hexahydroxylated Anthracene System", THE JOURNAL OF ORGANIC CHEMISTRY, vol. 71, no. 7, 1 March 2006 (2006-03-01), pages 2899-2902, XP055079452, ISSN: 0022-3263, DOI: 10.1021/jo052599x
- PIOTR BALCZEWSKI ET AL: "First Approach to Nitrogen-Containing Fused Aromatic Hydrocarbons as Targets for Organoelectronics Utilizing a New Transformation of O -Protected Diaryl Methanols", CHEMISTRY - A EUROPEAN JOURNAL, vol. 16, no. 8, 22 February 2010 (2010-02-22), pages 2392-2400, XP055079458, ISSN: 0947-6539, DOI: 10.1002/chem.200901947
- ANTONIO DE LA HOZ ET AL: "Microwaves in organic synthesis. Thermal and non-thermal microwave effects", CHEMICAL SOCIETY REVIEWS, vol. 34, no. 2, 1 January 2005 (2005-01-01), page 164, XP055079549, ISSN: 0306-0012, DOI: 10.1039/b411438h
- Timothy J Mason: "Ultrasound in synthetic organic chemistry", Chem. Soc. Rev., 1 January 1997 (1997-01-01), pages 443-451, XP055462180, DOI: 10.1039/CS9972600443 Retrieved from the Internet: URL:http://pubs.rsc.org/en/content/article pdf/1997/CS/CS9972600443 [retrieved on 2018-03-22]

## Description

### Technical Field

The subject of the invention is a new way of preparation of polycyclic, fused (hetero)aromatic hydrocarbons, presented in the formula 1, 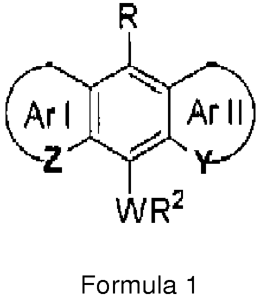 based on transformation reaction of derivatives with chemical formula 2 or 3, obtained from intermediates of formula 4 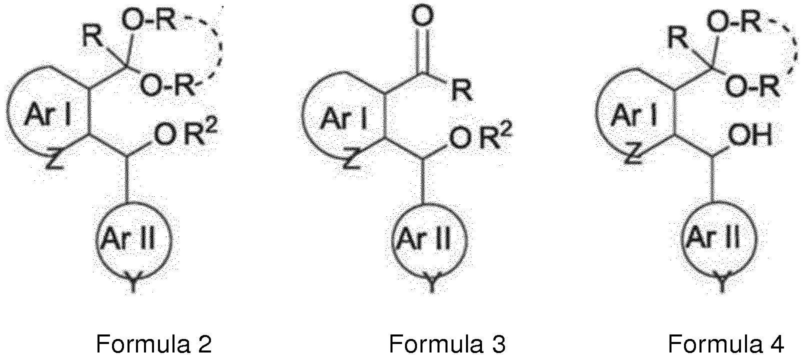 in water or an organic solvent, or a mixture of water and an organic solvent, or without an organic solvent in presence of an acidic reagent, with participation of ultrasounds, carried in normal pressure and room temperature, in a single reaction vessel, without a need of separation of intermediate products and with a substantial decreasing of the reaction time from 60-72 hrs to 15 minutes in comparison to procedures describes in applications P-385794 of 31.07.2008 and P-389778 of 05.12.2009.

The subject of the invention are also improvements involving a decrease of reaction times in reactions leading to formation of intermediates 2 and 3 in higher yields and better purities, by replacement of benzene for toluene, application of the brine washing during workup and reduction of the reaction time, in comparison to the protocols known from applications P-385794 of 31.07.2008 and P-389778 of 05.12.2009.

The subject of the invention are also improvements involving an increase of yields of compounds 4 by washing of the resulting post-reaction mixture with brine and simplification of the purification step by use of crystallization instead of chromatography without loss of yields, in comparison to the protocol known from applications P-385794 of 31.07.2008 and P-389778 of 05.12.2009.

The subject of the invention is also introduction of a combination of two or more acidic reagents of the same (Bronsted acid/Bronsted acid) or different types (Bronsted acid/Lewis acid) or occurring in the same (liquid/liquid or solid/solid) or different phases (acetic acid and gaseous HCl).

### Background Art

The inventive method involves use of intermediate compounds with chemical formulas 2, 3 and 4, presented in the Chart I. Compounds 2 are obtained from compounds 4, where:
W = oxygen or sulfur
Z and Y - are the same or different and stand for atoms of carbon or sulfur,
R - stands for hydrogen,
R2 - stands for alkyl, allyl,
Arl and ArII - are the same or different and stand for an aromatic ring, chosen from benzene, naphthalene, anthracene, phenanthrene, or pyrene, or a five-membered heteroaromatic ring chosen from pyrrole, thiophene, furan, or six-membered heteroaromatic ring chosen pyridine, pyrimidine, pyran, thiopyran, or a mixed aromatic-heteroaromatic ring chosen from indole, benzothiophene, or benzofuran; mono- or multi-substituted with alkyl group containing 1 to 20 atoms of carbon, allyl group, benzyl group, aryl group, with dialkylamine or diarylamine group , halide groups F, Cl, Br, I, ketone, aldehyde, acyl, carboxyl, imine, alkoxy, aryloxy, nitrile, nitro, and/or heteroaryl groups,
R---R stands for a cyclic acetal derivative

The method of preparation of polycyclic, fused (hetero)aromatic hydrocarbons presented by the formula 1, according to the invention, constitutes a previously unknown modification of intramolecular Friedel-Crafts type cyclization. The reaction uses *ortho-acetal* diarylmethanols, presented by the formula 2 or ortho-formyl diarylmethanols, presented by the formula 3, which in acidic environment undergo transformation to polycyclic, fused (hetero)aromatic hydrocarbons. Compounds 2 are obtained from compounds 4, and compounds 3 may be preferably obtained from compounds 2.

Literature mentions examples of intramolecular Friedel-Crafts reactions giving polycyclic, fused (hetero)aromatic hydrocarbons using ortho-formyl (M. S. Newman, N. S. Hussain, J. Org. Chem. 1982, 2837-2840) *ortho-*acyl (M. B. Andrus, Z. Ye, J. Zhang, Tetrahedron Lett.2005, 46, 3839-2842) and ortho-carboxyl (R. G. Harvey, C. Cortez, T. Sugiyama, Y. Ito, T. W. Sawyer, J. DiGiovanni, J. Med Chem. 1988, 31, 154-159) substituted diaryl methans and ortho-carboxyl (J. L. Hallman, R. A. Bartach, J. Org. Chem. 1991, 56, 6243-6245, K. L. Platt, F. Oesch, J. Org. Chem.1981, 2601-2603) substituted diaryl ketones. Except for the first two examples and the modification presented here, in those types of intramolecular cyclization an additional step of reduction of an intermediate product - respective anthrone or antraquinone - is required. Those reactions require also an unfavourable use of excessive acidic reagent at high concentration, and heating of the reaction mixture to a high temperature (R. Sangaiah, A. Gold, G. E. Toney, J. Org. Chem., 1983, 48, 1632-1638, M.. S. Newman, V. S. Prabhu, S. Veeraraghavad, J. Org. Chem. 1983, 48, 2926-2928; H. Ihmels, A. Meiswinkel, C. J. Mohrschladt, D. Otto, M.Waidelich, M. Towler, R. White, M. Albrecht, A. Schnurpfeil, J. Org. Chem. 2005, 70, 3929-3938). The best known example of Friedl-Crafts intramolecular cyclization is the Bradsher reaction (C. K. Bradsher, J. Am. Chem. Soc., 1940, 62, 486-488; C. K. Bradsher, F. A. Vikgiello, J. Org. Chem. 1948, 13, 786-789; C. K. Bradsher, Chem. Rev. 1987, 87, 1277-1297). Substrates of the reaction are ortho-formyl diarylmethanes, which in presence of 48% HBr, in boiling acetic acid undergo cyclization to polycyclic, fused (hetero)aromatic hydrocarbons.

Polycyclic, fused aromatic and heteroaromatic hydrocarbons presented by the general formula 1, where Z, Y, R, R2, Arl, ArII are as defined above, are known from applications P-385794 of 31.07.2008 and P-389778 of 05.12.2009, along with a method of production of those polycyclic, fused (hetero)aromatic hydrocarbons, based on transformation of intermediate compounds, in acidic environment, under mild conditions, in a single reaction vessel, and without separation of intermediate products.

Polycyclic, fused aromatic and heteroaromatic hydrocarbons presented by formulas 1a-1h may be used in construction of new generation of optoelectronic elements and devices, including organic, multi-layer light emitting diodes (OLEO and PLED), organic field effect transistors (OTFT, OFET), organic light emitting transistors (OLET), organic photovoltaic cells (OPV), organic solar cells (OSC), organic lasers, optical memories and other devices using organic semi- and photo-conductors *(*Highly Efficient OLEDs with Phosphorescent Materials, H.,Yersin (Ed.), Wiley-VCH Verlag GmbH & Co. KGaA, 2008, Introduction to Organic Electronic and Optoelectronic Materials and Devices, Sam-Shajing Sun, LR.Dalton (Edits), CRC Press, Taylor & Francis Group, Boca Raton, London, New York, 2008; Organic Field Effect Transistors, Z.Bao, J.Locklin (Edits), CRC Press, Taylor & Francis Group, Boca Raton, London, New York, 2007).

Due to their high photoluminescence and electroluminescence properties and thermal stability, anthracene and its derivatives are extensively studied as materials for construction of blue electroluminescence diodes (Y.-H. Kim, D.-C. Shin, S.-H. Kim, C.-H. Ko, H.-S. Yu, Y.-S. Chae, S.-K. Kwon, Adv. Mater., 2001, 13, 1690; W.-J. Shen, C. H. Chen, Tetrahedron Letters,2003, 44, 5747; Y.-H. Kim, H.-C. Jeong, S.-H. Kim, K. Yang, S.-K. Kwon, Adv. Funct. Mater., 2005, 15, 1799; B. Balaganesan, Y.-Y. Lyu, J. Kwak, 0. Kwon, S.-H. Lee, D. Kim, C. Lee, K. Char, Adv. Mater., 2008, 20, 2720; J. K. Park, K. H. Lee, S. Kang, J. Y. Lee, J. S. Park, J. H. Seo, Y. K. Kim, S.S. Yoon, Organic Electronics, 2010, 11, 905).

Also carbazole derivatives have been used in construction of organic field effect transistors (OFET) (Y. Li, Y. Wu, S. Gardner, B. S. Ong, Adv. Mater. 2005, 17, 849; N.-X. Hu, S. Xie, Z. Popovic, B. Ong, A.-M. HorSuning Wang, J. Am. Chem. Soc.1999, 121, 5097) and of organic electroluminescence diodes (S. S. Palayangoda, X. Cai, R. M. Adhikari, D.C. Neckers, Org. Lett 2008, 10, 281; V. Promarak, A. Punkvuang, T. Sudyoadsuk, S. Jungsuttiwong, S. Saengsuwan, T. Keawin, K. Sirithip, Tetrahedrorf2.007, 63, 8881). Polycyclic, fused heteroaromatic hydrocarbons 1 may also demonstrate a broad spectrum of biological effects. Literature mentions numerous examples of benzocarbazole derivatives (e.g. staurosporine, rebecamycin) demonstrating DNA intercalating properties (Leonard., J., Nat. Prod Rep., 1999, 16,319). Examples of other benzocarbazole derivatives are known (Bourderioux, A.; Routier, S.; Beneteau, V.; Merour, J.-Y. Tetrahedron Lett, 2005, 46, 6071), which act as enzyme inhibitors.

### Summary of invention

According to the invention, method of preparation of polycyclic, fused aromatic and heteroaromatic hydrocarbons with the formula 1, 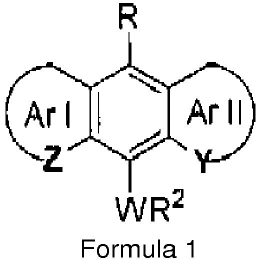 where
W = oxygen or sulfur
Z and Y - are the same or different and stand for atoms of carbon or sulfur,
R - stands for hydrogen,
R2 stands for alkyl, allyl,
ArI and ArII - are as defined above,
characterized by that, compounds with the formula 2, or with the formula 3 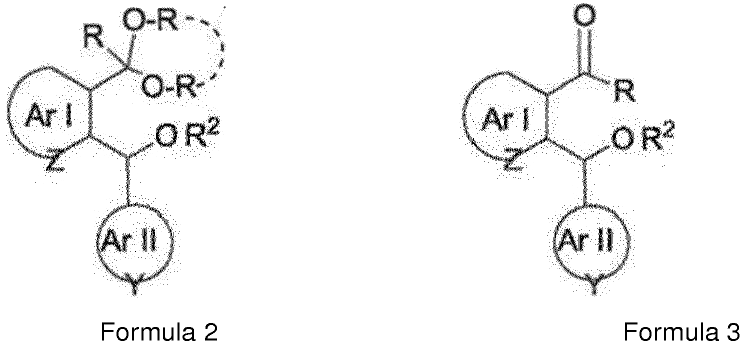 wherein
   Z, Y, R, R2, R---R, ArI, ArII are defined as above,
   are transformed by an intramolecular cyclization reaction to compounds of formula 1 in a reaction time of up to 3 hrs, whereby the reaction of transformation being carried out with the use of ultrasounds at room temperature or up to 60°C, in acidic environment, in a single reaction vessel, without separation of intermediate products.

As acidic reagents are used inorganic acids in any concentrations, gaseous HCl, organic acids, strong acidic ion-exchange resins, Lewis acid salts.

The inorganic acids are selected from the group consisting of HCl, HBr, HI, HF, H₂SO4, H₃PO₄.

The organic acids are selected from the group consisting of acetic, trifluoroacetic, methanesulfonic, trifluoromethanesulfonic, p-toluene-sulfonic.

The strong acidic ion-exchange resins are selected from the group consisting of Amberlite® IR 120, Amberlyst® 15.

The Lewis' acids are selected from the group consisting of FeCl₃, ZnCl₂, SnCl₂·H₂O, SnCl₄.

According to the invention the reaction is carried out using a combination of two or more acidic reagents of the same or different types or occurring in the same or different phases.

As a solvents are used water, mixtures of water-miscible organic solvents in any ratios, or organic solvents alone.

Preferably according to the invention the transformation reaction of compounds 2 or 3 is carried out, in water and with use of a water-miscible organic solvents in any ratios, or organic solvents alone, selected from the group consisting of alcohols, ketones, ethers, alcohol ethers, aprotic solvents.

The alcohols are selected from the group consisting of methanol, ethanol, n-propanol, iso-propanol, n-butanol, ethylene glycol, 1,3-propandiol, glycerol, 3-oxapentano-1,5-diol.

The ketones are selected from the group consisting of acetone, ethyl methyl ketone.

The ethers are selected from the group consisting of 1,2-dimethoxyethane (DME), tetrahydrofuran (THF), 1,4-dioxane, dimethyl ether of ethylene glycol. The alcohol ether is monomethyl ether of ethylene glycol.

The aprotic solvents are selected from the group consisting of, dimethylformamide (DMF), formamide, dimethyl sulfoxide (DMSO), hexamethylphosphoramide (HMPA), sulfolane, acetonitrile.

According to the invention the compounds with the formula 2 are obtained from compounds with formula 4 by protection of the hydroxyl group in the compounds 4 within 12 hours, wherein the variables are as defined as above.

According to the invention the compounds with the formula 3 are obtained from reaction of deacetalization of the compounds 2 in boiling toluene, in the presence of 1N HCl, within 12 hours, and the post-reaction mixture is salted out with saturated sodium chloride solution. Use of toluene instead of benzene results in a decrease of the deacetalization reaction time compared to the procedure reserved in the application P-389778 and leads to products with the formula 3 in quantitative yields. This protocol does not require purification, as it is in case of the application P-389778.

According to the invention the compounds with the formula 4 are obtained in the way involving reactions of protected orto-bromo aldehydes 5, wherein Hal is bromo, K and L are oxygen, with aromatic or heteroaromatic aldehydes 7, wherein the variables are as defined as above in the presence of n-BuLi, and salting out post-reaction mixtures with saturated sodium chloride solution.

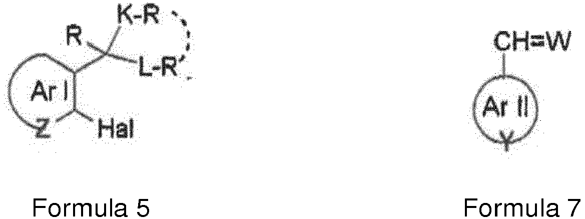

Compared to procedures reserved in applications P-389778 and P-385794, according to the inventive method, compounds with formula 4 are formed in very high and qualitative yields, despite their purification by simple crystallization, and not by column chromatography, as it is in case of applications P-389778 and P-385794.

### Description of embodiments.

Examples of realization of the invention are presented below.

### Example I (ultrasounds, water)

To a compound **2** (1 mmol) or a compound **3,** aqueous HCl solution of any concentration (e.g 0.1%, 1N, etc.) was added. Ultrasound probe was immersed in the resulting solution and the reaction was sonicated from several minutes to 3 hours, depending on the substrate used. Then, ethyl acetate was added and the organic layer was washed with water, NaHCO₃, water again, dried over MgSO₄ and evaporated. The crude product was purified by column chromatography on silica gel. Compounds **1a, 1b, 1c** were obtained in yields 52% for **1a,** 30% for **1b,** 64% for **1c.**

### Example II (ultrasounds, water/solvent)

A compound **2a** (1 mmol) or a compound **3a** (1 mmol) was dissolved in a water- miscible solvent (e.g. MeOH) (10 ml) and aqueous HCl solution (10 ml) of any concentration (e.g. 0.1%, 1N, etc.) was added. Ultrasound probe was immersed in the resulting solution and reaction was sonicated for 110 min. Then, ethyl acetate was added and the organic layer was washed with water, NaHCO₃, water again, dried over MgSO₄ and evaporated. The crude product was purified by column chromatography on silica gel. The compound **1a** was obtained in yield 62%.

### Example III (ultrasounds, water/solvent)

A compound **2a** (1 mmol) or a compound **3a** (1 mmol) was dissolved in a water-miscible solvent (e.g. MeOH) (10 ml) and aqueous HCl solution (10 ml) of any concentration (e.g. 0.1%, 1N, etc.) was added. Ultrasound probe was immersed in the resulting solution and reaction was sonicated for 110 min. Then, ethyl acetate was added and the organic layer was washed with water, NaHCO₃, water again, dried over MgSO₄ and evaporated. The crude product was purified by column chromatography on silica gel. The compound **1a** was obtained in a good yield (62%).

### Example IV (ultrasounds, solvent, ion-exchange resin)

To a solution of compounds **2a, 2c** (1 mmol) or **3a** (1 mmol) in an organic solvent (e.g. MeOH) strong acidic ion-exchange resin e.g. Amberlite® 120-type was added. Ultrasound probe was immersed in the solution and reaction was sonicated for 20 min. Then, ethyl acetate was added and the organic layer was washed with water, NaHCO₃ and water again. After drying over MgSO₄ and evaporation of solvents, the product was purified by column chromatography on silica gel. Compounds **1a** and **1c** were obtained in yields 41% for **1a,** 43% for **1c.**

### Example V (ultrasounds, 100% acid)

Compounds **2a** (1 mmol) or **3a** (1 mmol) were dissolved in acid (e.g. CH₃COOH) (1 mmol) and the resulting solution was sonicated for 15 minutes. After pouring the mixture on water, saturation with NaCl, ethyl acetate was added and the organic layer was washed with water, NaHCO₃ and water once again. Then, the organic layer was dried over MgSO₄, evaporated and the product was purified by column chromatography on silica gel. The compound **1a** was obtained in yield approx. 40%.

Spectroscopic data of the obtained compounds are presented in **Table 1.**

**Table 1**

| Compound | **¹H NMR [ppm] (200MHz, C₆D₆)** | **¹³C NMR [ppm] (50MHz, C₆D₆)** | **MS (EI/70eV or CI/isobutane** |
|---|---|---|---|
| **1a** | 3.50 (s, 3H, OCH₃), 3.75 (s, 3H, OCH₃), 3.86 (s, 3H, OCH₃), 3.94 (s, 3H, OCH₃), 5.27 (s, 2H, OCH₂O), 7.16 (s, 1H, Ar-H), 7.31 (s, 1H,Ar-H), 7.68 (s, 1H, Ar-H), 8.46 (s, 1H, Ar-H); | 55.92 (s,OCH₃), 61.75 (s, OCH₃), 62.05 (s,OCH₃), 96.41 (s,C_{Ar}H), 98.12(s,C_{Ar}H), 101.55 (s, OCH₂O), 104.53 (s, C_{Ar}H), 116.39 (s, C_{Ar}H), 122.80 (s, C_{Ar}), 123.44 (s, C_{Ar}), 125.72 (s, C_{Ar}), 130.29 (s, C_{Ar}), 131.31 (s, C_{Ar}), 134.29 (s, C_{Ar}), 141.89 (s, C_{Ar}); 146.52 (s, C_{Ar}), 148.95 (s, C-OCH₂O), 149.22 (s, OCH₂O-C); | 342 [M⁺¹, 100]; |
| **1a'** | 0.88-0.95 (m, 4H, OCH₂CH₂CH₂CH₃), 1.56 3H, OCH₂CH₂CH₂CH₃), (t, 2H, OCH₂CH₂CH₂CH₃) 3.57 (s, 3H, OCH₃), 3.88 3H, OCH₃), 3.96 (s, 3H OCH₃), 5.28 (s, 2H, OCH₂O), 7.16 (s, 1H, Ar-H), 7.41 (s, 1H, Ar-H), 7.77 (s, 1H, Ar-H), (s, 1H, Ar-H); | 12.51 (s,O(CH₂)₃CH₃), 18.19 (s, OCH₂CH₂CH₂CH₃), 31.40 (s, OCH₂CH₂CH₂CH₃), 53.56 (s, OCH₃), 59.27 (s, OCH₃), 59.43 (s, OCH₃), 72.74 (s, OCH₂CH₂CH₂CH₃), 94.35 (s, C_{Ar}H), 95.96 (s, C_{Ar}H), 99.21 (s, C_{Ar}H), 102.20 (s, OCH₂O), 113.90 (s, C_{Ar}H), 120.79 (s, C_{Ar}), 127.00 (s, C_{Ar}), 146.25 (s, C-OCH₂O), 146.82 (s, OCH₂O-C), 151.82 (s, C_{Ar}); | 384 [M⁺, 35], 327 [M⁺,-(CH₂)₃CH₃, 100]; |
| **1a"** | 0.85-0.91 (m, 3H, OCH₂(CH₂)₁₄CH₃), 1.21-1.35 (m, 28H, OCH₂(CH₂)₁₄CH₃), 3.18 (t, 2H, OCH₂(CH₂)₁₄CH₃), 4.00 (s, 6, OCH₃), 4.02 (s, 3H, OCH₃), 5.96 (s, 1H, OCH₂O), 3.04 (s, 1H, OCH₂O), 6.96 (s, 1H, Ar-H), 7.17-7.23 (m, 2H, Ar-H), 8.15 (s, 1H, Ar-H); | 7.28 (s, CH₂), 14.10(s,CH₂), 22.67(s, CH₂) 29.67 (s, CH₂), 57.71 (s, OCH₃), 61.31 (s,OCH₃), 82.88(s, OCH₂(CH₂)₁₄ CH₃), 95.32 97.07(s, C_{Ar}H), 101.79 (s, OCH₂O) 107.49 (s, C_{Ar}H), 114.65 (s, C_{Ar}H), 120.79 (s C_{Ar}), 127.10 (s, C_{Ar}), 128.42 (s, C_{Ar}), 139.52 (s C_{Ar}), 140.14 (s, C_{Ar}), 147.31 (s, C-OCH₂O) 147.50 (s, OCH₂O-C), 152.44 (s, C_{Ar}); | 553 [M⁺¹, 27], 327 [M⁺¹, - CH₂(CH₂)₁₄ CH₃, 100]; |
| **1b** | 3.48 (s, 3H, OCH₃), 3.74 (s, 3H, OCH₃), 5.28 (s, 2H, OCH₂O), 7.09 (s, 1H, Ar-H), 7.23-7.25 (m, 2H, Ar-H), 7.60-7.69 (m, 1H, Ar-H); | | 300 [M⁺, 100], 285 [M⁺, - Me, 40], 286 [M⁺, -MeOH, 45] |
| **1c** | 4.03 (s, 3H, CH₃), 3.17 (s, 3H, CH₃), 6.06 (s, 2H, OCH₂O), 6.99-7.03 (m, 2H, Ar-H), 7.23-7.38 (m, 3H, Ar-H), 7.48-7.51 (m, 2H, Ar-H), 8.09-8.16 (m, 2H, Ar-H); | 31.41 (s, CH₃), 62.82 (s, CH₃), 98.13 (s, OCH₂O), 96.93 (s, C_{Ar}-H), 100.87 (s, C_{Ar}-H), 104.00 (s, C_{Ar}-H), 108.20 (s, C_{Ar}-H),113.83 (s, C_{Ar}-H), 118.90 (s, C_{Ar}-H), 120.32 (s, C_{Ar}-H), 122.97 (s, C_{Ar}), 124.01 (s, C_{Ar}), 125.74 (s, C_{Ar}), 125.74 (s, C_{Ar}), 126.71 (s, C_{Ar}-H), 127.59 (s, C_{Ar}-H), 128.14 (s, C_{Ar}-H), 128.67 (s, C_{Ar}H),), 131.34 (s, C_{Ar}), 137.08 (s, C_{Ar}), 137.17 (s, C_{Ar}), 143.61 (s, C), 145.76 (s, C), 147.62 (s, C); | 305 [M⁺¹, 57], 290 [M⁺¹, -Me, 100]; |
| **1c'** | 3.56 (s, 3H, N-CH₃), 4.01 (s, 2H, CH₂Br), 4.72 (s, 2 H, OCH₂Ph), 5.38 (s, 2H, OCH₂O), 6.82 (s, 1H, Ar-H), 7.01-7.44 (m, 5H, ArH), 7.81 (s, 1H, Ar-H), 8.10-8.12 (m, 2H, Ar-H); | 31.73 (s, CH₃), 76.76 (s, OCH₂Ph), 98.05 (s, OCH₂O), 101.67 (s, C_{Ar}H), 105.30 (s, C_{Ar}H), 109.31 (s, C_{Ar}H), 115.21 (s, C_{Ar}H), 120.07 (s, C_{Ar}H), 121.51 (s, C_{Ar}H); 124.29 (s, C_{Ar}H), 125.33 (s, C_{Ar}H), 127.13 (s, C_{Ar}H), 130.18 (s, C), 132.49 (s, C), 138.50 (s, C_{Ar}H), 144.84 (s, C), 147.16 (s, C); | 474 (5) [M⁺¹ (79),], 476 (4)(M⁺¹(81)], 396 (10) [M⁺¹-Br], 306 (30) [M⁺¹-BrCH₂Ph], 292 (100) [M⁺¹-BrCH₂PhCH₂]. |
| **1d** | 3.06 (s, 3H, OCH₃), 3.28 (s, 6H, OCH₃), 3.84 (s, 3H, OCH₃), 6.00 (s, 1H, OCHO), 5.88 (s, 1H, CHOMe), 6.69 (s, 2H, ArH), 6.95-6.99 (m, 2H, ArH), 7.28 (d, 1H, ArH), 8.00 (d, 1H, ArH), 10.64 (s, 1H, CHO); | *56.43* (s, CH₃), 56.44 (s, CH₃), *61.07*(s, CH₃), 80.82 (s, CHOMe), 105.39 s, C_{Ar}H), 124.15 (s, C_{Ar}H), 125.69 (s, C_{Ar}H), 26.74 (s, C_{Ar}H), 136.36 (s, C_{Ar}), 139.34 (s, C_{Ar}), 139.89 (s, C_{Ar}), 141.74 (s, C_{Ar}), 143.20 s, C_{Ar}), 145.06 (s, C_{Ar}), 154.84 (s, C_{Ar}-H), 85.50 (s, CHO); | 355 [M⁺¹, 91], 354 [M⁺, 100], 339 [M⁺, -Me, 65]; |
| **1e** | 5.35 (s, 2H, OCH₂Ph), 7,44 (d, 2H, Ar); 7.50-7.78 (m, 5H, C₆H₅); 7.88 (m, 1H, Ar); 8.25 (m, 1H, Ar), 8,42 (s, 1H, Ar), 8,51 (s, 1H, Ar), 8,65 (s, 1H, Ar), 10.52 (s, 1H, CHO). | 75.34 (s, OCH₂Ph); 118.11 (s, C_{Ar}H); 120.21 (s, C_{Ar}H); 122.44 (s, C_{Ar}H); 123.04 (s, C_{Ar}H); 125.20 (s, C_{Ar}H); 126.26 (s, C_{Ar}H); 128.50 (s, 2x *o*-Ph); 128.74 (s, *p*-Ph); 129.88 (s, 2x *m-*Ph); 130.76 (s, C_{Ar}); 133.71 (s, C_{Ar}), 134.02 (s, C_{Ar}), 135.32 (s, C_{Ar}), 139.06 (s, C_{Ar}), 140, 33 (s, C_{Ar}), 142,11 (s, C_{Ar}) 143.67 (s, C_{Ar}), 153.88 (s, C_{Ar}), 155.09 (s, C_{Ar}), 191.89 (s, CHO). | 382 (M, 26); 305.9 (M(-Ph), 100) |
| **1f** | 5.37 (s, 2H, OCH₂Ph), 7,49 (d, 2H, Ar); 7.49-7.70 (m, 5H, C₆H₅); 7.89 (m, 1H, Ar); 8.23 (m, 1H, Ar), 8,45 (s, 1H, Ar), 8,49 (s, 1H, Ar), 8,70 (s, 1H, Ar), 10.60 (s, 1H, CHO). | 76.40 (s, OCH₂Ph); 120.16 (s, C_{Ar}H); 121.33 (s, C_{Ar}H); 123.03 (s, C_{Ar}H); 123.44 (s, C_{Ar}H); 126.27 (s, C_{Ar}H); 126.56 (s, C_{Ar}H); 128.59 (s, 2x *o*-Ph); 128.84 (s, *p*-Ph); 129.23 (s, 2x *m-*Ph); 133.76 (s, C_{Ar}); 134.67 (s, C_{Ar}); 134.82 (s, C_{Ar}); 135.22 (s, C_{Ar}), 139.47 (s, C_{Ar}), 142.76 (s, C_{Ar}), 146,45 (s, C_{Ar}), 149,55 (s, C_{Ar}) 155.28 (s, C_{Ar}), 156.37 (s, C_{Ar}); 190.19 (s, CHO). | 382 (M, 33); 305.9 (M(-Ph), 100.00) |
| **1g** | 4.11 (s, 3H, CH₃); 5.17 (s, 2H, OCH₂Ph); 7.06-7.30 (m, 8H, C₆H₅, Ar-H); 8.42 (d, 1H, Ar); 8.46 (d, 2H, Ar); 8.65 (s, 1H, Ar) 10.48 (s, 1H, CHO). | 31.47 (s, CH₃), 79.10 (s, OCH₂Ph), 108.75 (s, C_{Ar}H); 117.71 (s, C_{Ar}H); 119.50 (s, C_{Ar}H);120.23 (s, C_{Ar}H) 121.11 (s,C_{Ar}H), 122.52 (s, C_{Ar}H), 125.35 (s, C_{Ar}H),126.35 (s, C_{Ar}), 127.67 (s, C_{Ar}), 127.94 (s, C_{Ar}), 128.32 (s,C_{Ar}), 128,36 (s, C_{Ar}), 128.61 (s, 2x *o*-Ph); 128.70 (s, *p*-Ph); 128.84 (s, 2x *m*-Ph); 129.88 (s, C_{Ar-}Br), 133.23 (s, *ipso*-C₆H₅), 136.09(s, C_{Ar}), 136.35(s, C_{Ar}), 144,30 (s, C-OCH₂Ph), 191.91 (s, CHO). | 43(M,20.00); 353(M(-CH₂Ph), 100) |
| **1h** | 4.10 (s, 3H, CH₃); 5.20 (s, 2H, OCH₂Ph); 7.04-7.30 (m, 8H, C₆H₅, Ar-H); 8.44 (d, 1H, Ar); 8.46 (d, 2H, Ar); 8.67 (s, 1H, Ar) 10.50 (s, 1H, CHO). | 31.45 (s, CH₃), 80.10 (s, OCH₂Ph), 109.15 (s, C_{Ar}H); 116.81 (s, C_{Ar}H); 118.90 (s, C_{Ar}H); 120.23 (s, C_{Ar}H) 121.15 (s, C_{Ar}H), 123.02 (s, C_{Ar}H), 126.35 (s, C_{A}rH),127.30 (s, C_{Ar}), 127.68 (s, C_{Ar}), 127.96 (s, C_{Ar}), 127.28 (s, C_{Ar}), 127.33 (s, C_{Ar}), 128.61 (s, 2x *o*-Ph); 128.70 (s, *p*-Ph); 129.84 (s, 2x *m*-Ph); 129.81 (s, C_{Ar}Br), 137.28 (s, *ipso*-C₆H₅), 136.07(s, C_{Ar}), 138.38 (s, C_{Ar}), 145,31 (s, C-OCH₂Ph), 192.92 (s, CHO). | 443 (M, 25); 353 (M-(PhCH₂), 100) |

### Example VI. General procedure for synthesis of compounds with the formula 2 (improvement by reduction of the reaction time).

To NaH suspension (1.1 mmol) in dry THF a solution of the compound with the formula **4** (1.2 mmol) in dry THF was gradually added. The mixture was stirred for 30 minutes at room temperature, and then alkyl halide was added, and left for 12 hours at room temperature. After evaporation of the solvent, ethyl acetate was added and the mixture was washed twice with water. The organic layer was dried over MgSO₄ and then evaporated to give compounds with formulas **2a-2h** in quantitative yields.

Spectroscopic data of the obtained compounds are presented in **Table 2.**

**Table 2**

| Compund | ¹H NMR [ppm] (200MHz, C₆D₆) | ¹³C NMR [ppm] (50MHz, C₆D₆) | MS (EI/70eV or CI/isobutune |
|---|---|---|---|
| **2a** | 0.73-0.79(m,1H,5-H_{eq}), 1.74-1.95 (m,1H,5-Hₐₓ), 3.35(s,3H,OCH₃), 3.37-3.50(m,2H,4-Hₐₓ,6-Hₐₓ), 3.45 (s,6H,OCH₃), 3.75-3.90 (m, 2H,4-H_{eq},6-H_{eq}), 3.85(s,3H,OCH₃), 5.17 (s,1H,OCH₂O),5.22(s,1H, OCH₂O), 5.67 (s, 1H, OCHO), 5.88 (s, 1H, CHOMe), 6.99 (s, 2H, Ar-H), 7.19 (s, 1H, Ar-H), 7.50 (s, 1H, Ar-H); | 26.48 (s,OCH2CH₂CH₂O), 56.46(s,OCH₃), 57.21 (s, OCH₃), 61.15 (s, OCH₃), 67.89 (s, OCH₂CH₂CH₂O), 68.02(s,OCH₂CH₂CH₂O), 30.79 (s, CHOMe), 100.91 (s, OCH₂O), 101.85 (s,OCHO), 105.51 (s,C_{Ar}-H),108.02 (s, C_{Ar}), 108.80(s, C_{Ar}), 132.59 (s, C-CHO(CH₂)₃O), 135.50 (s, C-CHOMe), 139.23 (s, C_{Ar}), 148.25 (s, C-OCH₂O), 149.47 (s, OCH₂O-C), 154.58 (s, C_{Ar}-H) ; | 418[M⁺,30], 387[M⁺,-OMe,100],3 42 [M⁺, - HO(CH₂)₃O, 70]; |
| **2a'** | 0.73-0.79 (m, 1H,5-H_{eq}), 0.83-0.92 (m, 4H, OCH₂CH₂CH₂CH₃), 1.45 (t, 3H, CH₂CH₂CH₂CH₃), 3.56 (t, 2H, OCH₂CH₂CH₂CH₃), 3.31-3.47 (m, 2H, 4-Hₐₓ, 6-Hₐₓ), 3.47 (s, 6H, 2x OCH₃), 3.73-3.93 (m, 2H, 4-H_{eq}, 6-H_{eq}), 3.84 (s, 3H, OCH₃), 5.21 (s, 1H, OCH₂O), 5.26 (s, 1H, OCH₂O), 5.73 (s, 1H, OCHO), 5.97 (s, 1H, CHOC₄H₉), 6.98 (s, 2H, Ar-H), 7.20 (s, 1H, Ar-H), 7.49 (s, 1H, Ar-H); | 14.86(s,O(CH₂)₃CH₃), 20.62(s,OCH₂CH₂ CH₂CH₃),26.52(S,OCH₂CH₂CH₂O), 33.20(s,OCH₂CH₂CH₂CH₃), 56.49(s,OCH₃), 61.18 (s,OCH₃), 67.95 (s,OCH₂CH₂CH₂O), 68.07 (S,OCH₂CH₂CH₂O), 69.56 (s, OCH₂(CH₂)₂CH₃), 79.40 (S,CHO(CH₂)₃CH₃), 100.98(s,OCH₂O), 101.86 (s,OCHO), 105.65 (s,C_{Ar}-H), 108.05(s,C_{Ar}), 108.83(s,C_{Ar}), 132.35(s,C-CHO(CH₂)₃O), 135.94(s,C-CHOMe), 139.51(s,C_{Ar}), 148.16(s,C-OCH₂O), 149.40 (s, OCH₂O-C), 154.72 (s, C_{Ar}-H); | 460 [M⁺, 30], 387 [M⁺,-O(CH₂)₃CH₃, 100], 342 [M⁺, - HO(CH₂)₃OH, 34]; |
| **2a"** | 0.70-0.80 (m, 1H,5-H_{eq}), 0.81-0.90 (m, 3H, OCH₂(CH₂)₁₄CH₃), 1.03-1.50 (m, 28H, OCH₂(CH₂)₁₄CH₃), 1.70-1.99 (m, 1H, 5-Hₐₓ), 3.48 (s, 6H, OCH₃), 3.44-3.52 (m, 2H, 4-Hₐₓ, 6-Hₐₓ), 3.49 (s, 6H, 2x OCH₃), 3.62 (t, 2H, OCH₂(CH₂)₁₄CH₃), 3.81-3.90 (m, 2H, 4-H_{eq}, 6-H_{eq}), 3.85 (s, 3H, OCH₃), 5.18 (s, 1H, OCH₂O),5.24 (s, 1H,OCH₂O), 5.75 (s, 1H, OCHO), 6.01 (s, 1H, CHOC₁₆H₃₃), 7.01 (s, 2H, Ar-H), 7.24 (s, 1H, Ar-H), 7.51 (s, 1H, Ar-H); | *7.46 (s, CH₂), 15.04* (s, CH₂), 23.79 (s, CH₂), 26.54 (s, OCH₂CH₂CH₂O), 33.01 (s, CH₂), 56.51 (s, OCH₃), 61.17 (s, OCH₃), 68.07 (s, OCH₂CH:CH₂O), 68.47 (s, OCH₂CH₂CH₂O), 69.94 (s, OCH₂(CH₂)₁₄CH₃), 79.45 (s, CHO(CH₂)₃CH₃), 100.91 (s, OCH₂O), 101.83 (s, OCHO), 105.71 (s, C_{Ar}-H), 108.05 (s, C_{Ar}), 108.83 (s, C_{Ar}), 132.37 (s, C-CHO(CH₂)₃), 135.96 (s, C-CHOC₁₆H₃₃), 139.49 (s, C_{Ar}), 139.92 (s, C_{Ar}), 148.17 (s, C-OCH₂O), 149.41 (s, OCH₂O-C), 154.76 (s, C_{Ar}-H); | 628 [M⁺, 32], 552 [M⁺, - HO(CH₂)₃O H, 38], 387 [M⁺, - O(CH₂)₁₅CH 3, 100]; |
| **2b** | 0.66-073 (m, 1H,5-H_{eq}), 1.86-1.91 (1H, m, 5-Hₐₓ), 3.09 (s, 3H, OCH₃), 3.29 (s, 3H, OCH₃), 3.45-3.52 (m, 2H, 4-Hₐₓ, 6-Hₐₓ), 3.83-3.90 (m, 2H, 4-H_{eq}, 6-H_{eq}), 5.17 (s, 1H, OCH₂O), 5.23 (s, 1H, OCH₂O), 5.93(s, 1H, OCHO), 7.30 (s, 1H, Ar-H), 7.35-7.39 (m, 1H, Ar-H), 7.66 (s, 1H, Ar-H), 7.82 (m, 1H, Ar-H); | 26.67 (s,OCH2CH₂CH₂O), 55.49 (s,OCH₃), 61.22 (s,OCH₃), 67.71 (s,OCH₂CH₂CH₂O), 67.84 (s,OCH₂CH₂CH₂O), 71.38 (s, OCH₂Ph), 99.77 (s,OCH₂O), 101.66 (s, OCHO), 107.67 (s, C_{Ar}-H), 108.65 (s, C_{Ar}-H), 111.33 (s, C_{Ar}-H), 121.87 (s, C_{Ar}-H), 128.01 (s, C_{Ar}-H), 132.21 (s, C_{Ar}), 132.94 (s, C_{Ar}), 134.41 (s, C_{Ar}), 149.00 (s, OCH₂O-C), 149.11 (s, C-OCH₂O), 157 (C_{Ar}-H); | 358 [M⁺, 16], 299 [M+, - O(CH₂)₂CH₃, 45], 282 [M⁺, - HO(CH₂)₃OH, 100] |
| **2c** | *0.53-0.60* (m, 1H,5-H_{eq}), 1.88-1.75 (1H, m, 5-Hₐₓ), 3.07-3.31 (m, 2H, 4-Hₐₓ, 6-Hₐₓ), 3.27 (s, 3H, CH₃), 3.39 (s, 3H, CH₃), 3.75-3.92 (m, 2H, 4-H_{eq}, 6-H_{eq}), 5.28-5.29 (m, 2H, OCH₂O), 5.61 (s, 1H, OCHO), 5.83 (s, 1H, CHOMe), 6.34 (s, 1H, ArH), 7.08-7.29 (m, 4H, Ind-H), 7.58-7.63 (m, 1H, Ind-H), 7.64 (s, 1H, ArH); | 26.50 (s, OCH:CH₂CH₂O), *30.60* (s, CH₃), 57.12 (s, CH₃), 67.73 (s, OCH₂CH₂CH₂O), 67.81 (s, OCH₂CH₂CH₂O), 76.58 (s, CHOMe), 100.18 (s, OCH₂)O), 101.94 (s, OCHO), 104.01 (s, C_{Ar}-H), 108.76 (s, C_{Ar}-H), 109.11 (s, C_{Ind}-H), 110.60 (s, C_{Ind}-H), 120.56 (s C_{Ind}-H,), 122.10 (s, C_{Ind}-H), 122.74 (s, C_{Ind}-H), 132.15 (s, C-CHOCH₂CH₂O), 133.08 (s, C-CHOMe), 139.52 (s, MeN-C_{Ar}), 140.44 (s, C_{Ar}-NMe), 148.60 (s, OCH₂O-C), 149.14 (s, C-OCH₂O); | 381 [M⁺, 100], 350 [M⁺,-OMe), 80], 306 [M⁺, - O(CH₂)₃OH, 40]; |
| **2d** | 0_{.}56-0_{.}62 (m, 1H,5-H_{eq}), 1.64-1.88 (1H, m, 5-Hₐₓ), 3.32 (s, 3H, OCH₃), 3.19-3.35 (m, 2H, 4-Hₐₓ, 6-Hₐₓ), 3.42 (s, 6H, OCH₃), 3.73-3.85 (m, 2H, 4-H_{eq}, 6-H_{eq}), 3.81 (s, 3H, OCH₃), 6.00 (s, 1H, OCHO), 6.02 (s, 1H, CHOMe), 6.97-7.12 (m, 2H, ArH), 7.09 (s, 2H, ArH), 7.48 (d, 1H, ArH), 8.18 (d, 1H, ArH); | *26_{.}17* (s, OCH₂CH₂CH₂O,), *56.45* (s, CH₃), 57.42 (s, CH₃), *61.11* (s, CH₃), 67.99 (OCH₂CH₂CH₂O), 79.85 (s, CHOMe), 98.49 (s,OCHO), 105.37 (s, C_{Ar}H), 123.40 (s, C_{Ar}H), 129.18 (s, C-CHO(CH₂)₃O), 133.81 (s, C_{Ar}), 136.77 (s, C_{Ar}), 139.21 (s, C_{Ar}), 139.24 (s, C_{Ar}), 139.68 (s, C_{Ar}), 140.66 (s, C_{Ar}), 154.84 (s, C_{Ar}-H); | 430 [M⁺, 100], 399 [M⁺, -MeOH, 60]; |
| **2e** | 1,25-1,47 (m, 2H, H_{eq}⁵, 5'), 1.96-2,39 (m, 2H, Hₐₓ^{5, 5'}), 3.68-3.81 (m, 2H, *H*ₐₓ^{4, 4', 6, 6'}), 3.98-4.20 (m, 4H, *H*_{eq}^{4, 4', 6, 6'}), 4.26-4.52 (m, 2H, *H*ₐₓ^{4, 4', 6, 6'}), 4.56-4,76 (d, 1H_{A}, ²J_{HH}=0.05 Hz, CH_{A}H_{B}Ph), 4.62-4,70 (d, 1H_{B}, ²J_{HH}=0,05 Hz, CH_{A}H_{B}Ph), 5.52 (s, 1H, OCHO), 5.81 (s, 1H, OCHO), 6.36 (s, 1H, CHOBn), 7.01 [s, 1H, Ar-H], 7.30-7.44 (m, 7H, Ph, Ar-H), 7.69 (t, 1H, Ar-H), 7.82 (t, 1H, Ar-H), 7.98 (s,1H,Ar-H), 8.00(s,1H,Ar-H). | 25.28 (s, OCH:CH₂CH₂O), 25.47 (s, OCH₂CH₂CH₂O), 67.00 (s, OCH₂CH₂CH₂O), 67.05 (s, OCH₂CH₂CH2O), 67.32 (s, OCH₂CH₂CH₂O), 70.43 (s, OCH₂Ph), 73.40 (s, CHOBn), 100.07(s, OCHO), 100.40 (s, OCHO), 121.75 (s, C_{Ar}-H), 122.01 (s, C_{Ar}-H), 123.04 (s, C_{Ar}-H),123.39 (s, C_{Ar}-H), 123.87 (s, C_{Ar}- H), 126.76 (s, C_{Ar}- H) 127.45 (s, *m-*Ph), 127.61 (s, *p*-Ph), 128.16 (s, *o*-Ph), 128.57 (s, C_{Ar}-H), 132.06 (s, C_{Ar}), 136.00 (s, C_{Ar}), 137.04(s, C_{Ar}), 137.91 (s, C_{Ar}), 139.32 (s, C_{Ar}), 139,70 (s, C_{Ar}), 141,23 (s,C_{Ar}), 146.51 (s, C_{Ar}) | 614 [M+2, 8], 431 [M, (OC₃H₆O), 100], 91 [(PhCH₂O), 76] |
| **2f** | 1,21-1,40 (m,2H,H_{eq}^{5, 5'}), 1.90-2,40 (m,2H,Hₐₓ^{5, 5'}), 3.45 (s, 1H, CH₃), 3.65-3.80 (m, 2H, *H*ₐₓ^{4, 4', 6, 6'}), 3.97-4.22 (m,4H,*H*_{eq}^{4, 4', 6, 6'}), 4.25-4.53 (m,2H, *H*ₐₓ^{4, 4', 6, 6'}), 4.55-4,75 (d, 1H_{A}, ²J_{HH}=0.06 Hz, CH_{A}H_{B}Ph), 4.61 -4,69 (d, 1H_{B}, ²J_{HH}=0,06 Hz, CH_{A}H_{B}Ph), 5.54 (s, 1H, OCHO), 5.84 (s,1H, OCHO), 6.33 (s,1H,CHOBn), 7.02 (s,1H,Ar-H), 7.29-7.43 (m, 7H, Ph, Ar-H), 7.70 (t, 1H, Ar-H), 7.80 (t,1H,Ar-H), 7.97(s,1H,Ar-H),8.02(s,1H,Ar-H | 25.31 (s,OCH₂CH₂CH₂O), 25.51 (s,OCH₂ CH₂CH₂O), 67.07 (s, OCH₂CH₂CH₂O), 67.09 (S,OCH₂CH₂CH₂O), 67.41 (s, OCH₂CH₂CH₂O), 70.53 (s, OCH₂Ph), 73.40 (s, CHOBn), 100.11 (s, OCHO), 100.45 (s, OCHO), 121.78 (s, C_{Ar}-H), 122.58 (s, C_{Ar}-H), 123.34 (s, C_{Ar}-H),123.43 (s, C_{Ar}-H), 123.99 (s, C_{Ar}-H), 126.70 (s, C_{Ar}-H),127.60 (s, m-Ph), 127.69 (s, *p*-Ph), 128.21 (s, *o*-Ph), 128.69 (s, C_{Ar}-H), 132.13 (s, C_{Ar}), 136.59 (s, C_{Ar}), 136.89 (s, C_{Ar}), 137.97 C_{Ar}), 139.34 (s, C_{Ar}), 139,79 (s,C_{Ar}), 142,22 (s, C_{Ar}), 146.58(s, C_{Ar}) | 614 [M+2, 5], 431 [M, (-OC₃H₆O-), 100], 91 [(PhCH₂O), 66] |
| **2g** | | 26.03 (s, OCH:CH₂CH₂O), 27.13 (s, OCH₂CH₂CH₂O), 30.08(s, CH₃), 66.04(s, CHOBn), 67.12(s,OCH₂CH₂CH₂O), 67.36 (s.OCH₂CH₂CH₂O), 67.41 (s,OCH₂CH₂CH₂O), 72.41 (s,OCH₂Ph), 100.36(s,OCHO), 100.81 (s, OCHO), 102.29 (s, C_{Ar}-H), 109.82 (s, C_{Ar}-H), 120.15 (s, C_{Ar}-H), 121.93 (s, C_{Ar}-H), 122.83 (s, C_{Ar}-H), 127.50 (s, C_{Ar}-H), 127.95 (s, *m*-Ph), 128.47 (s, *p*-Ph), 129.90 (s, *o*-Ph), 131.69 (s, C_{Ar}), 131.94 (s, C_{Ar}-H), 135.37 (s, C_{Ar}), 136.65 (s, C_{Ar}), 137,23 (s, C_{Ar}), 138.19 (s, C_{Ar}), 138.29 (s, C_{Ar}), 142.27 (s, C_{Ar}), 157.06 (s, C_{Ar}). | 578 [M+1, 7], 413[M,(-OC₃H₆O,PhCH₂O)70], 91 [M, (PhCH₂), 100] |
| **2h** | 1,25-1,43 (m, 2H, H_{eq}^{5, 5'}), 1.91-2,44 (m, 2H, Hₐₓ^{5, 5'}), 3.45 (s, 1H, CH₃), 3.64-3.83 (m, 2H, *H*ₐₓ^{4, 4', 6, 6'}), 3.99-4.23 (m, 4H, *H*_{eq}^{4, 4', 6, 6'}), 4.26-14.51 (m, 2H, *H*ₐₓ^{4, 4', 6, 6'}), 4.54-4,76 (d, 1H_{A}, ²J_{HH}=0.06 Hz, CH_{A}H_{B}Ph), 4.62-4,70 (d, 1H_{B}, ²J_{HH}=0,06 Hz, CH_{A}H_{B}Ph), 5.55 (s, 1H, OCHO), 5.83 (s, 1H, OCHO), 6.35 (s, 1H, CHOBn), 7.00 (s, 1H, Ar-H), 7.30-7.40 (m, 7H, Ph, Ar-H), 7.71 (t, 1H, Ar-H), 7.81 (t, 1H, Ar-H), 7.99 (s, 1H, Ar-H), 8.01 (s, 1H, Ar-H). | 26.83(s,OCH₂CH₂CH₂O), 27.16 (s,OCH₂₋CH₂CH₂O), 30.08 (s, CH₃), 66.00 (s, CHOBn), 67.02 (s, OCH₂CH₂CH₂O), 67.34 (s, OCH₂CH₂CH₂O), 67.51 (s, OCH₂CH₂CH₂O), 72.40 (s, OCH₂Ph), 100.25 (s, OCHO), 100.65 (s, OCHO), 101.13 (s, C_{Ar}-H), 106.54 (s, C_{Ar}-H), 118.19 (s, C_{Ar}-H), 121.34 (s, C_{Ar}-H), 123.24 (s, C_{Ar}-H), 126.21 (s, C_{Ar}-H), 128.16 (s, *m*-Ph), 128.42 (s, *p*-Ph), 130.90 (s, *o*-Ph), 132.91 (s, C_{Ar}-H), 134.60 (s, C_{Ar}), 134.71 (s, C_{Ar}), 135.22 (s, C_{Ar}), 135.75 (s, C_{Ar}), 138.91 (s, C_{Ar}), 139.09 (s, C_{Ar}), 142.69 (s, C_{Ar}), 157.16 (s, C_{Ar}). | 578 [M+1, 10], 413 [M, (-OC₃H₆O, PhCH₂O) 70], 61 [M, (PhCH₂), 100] |

### Example VII. General procedure for synthesis of compounds with the formula 3 (improvement by a benzene/toluene exchange and additional workup brine saturation).

To solution of a compound **2** in toluene, 1N HCl solution was added. The mixture was heated at the solvent boiling point. Then, ethyl acetate was added, the mixture was saturated with solid sodium chloride and organic layer was washed with water, NaHCO₃ solution, and with water once again. After drying over MgSO₄ and solvent evaporation, compounds presented by formulas **3a-d** were quantitatively obtained in a pure form.

Spectroscopic data of the obtained compounds are presented in **Table 3.**

**Table 3**

| **Compound** | **¹H NMR [ppm] (200MHz, C₆D₆)** | **¹³C NMR [ppm] (50MHz, C₆D₆)** | **MS (EI/70eV or CI/isobutane** |
|---|---|---|---|
| **3a'** | 0.79-0.87(m,4H, OCH₂CH₂CH₂CH₃), 1.37(t, 3H, OCH₂CH₂CH₂CH₃), 3.42 (s, 6H, OCH₃), 3.43 (t,3H, OCH₂CH₂CH₂CH₃), 3.79 (s, 3H, OCH₃), 5.13 (s, 1H, OCH₂O), 5.21 (s, 1H, OCH₂O), 6.27 (s, 1H, CHOC₄H₉), 6.82 (s, 2H, Ar-H), 7.20 (s, 1H, Ar-H), 7.29 (s, 1H, Ar-H), 10.13 (s, 1H, CHO); | 14.75 *(s,* O(CH₂)₃CH₃), 20.51 (s, OCH₂CH₂CH₂CH₃), 33.07 (s, OCH₂CH₂CH₂CH₃), 56.56 (s, OCH₃), 61.13 (s, OCH₃), 69.83 (s, OCH₂(CH₂)₂CH₃), 79.36 (s, CHO(CH₂)₃CH₃), 102.58 (s, OCH₂O), 105.79 (s, C_{Ar}-H), 108.95 (s, C_{Ar}), 111.18 (s, C_{Ar}), 138.39 (s, C_{Ar}), 139.73 (s, C-CHO(CH₂)₃CH₃), 143.20 (s, C_{Ar}), 148.43 (s, C-OCH₂O), 153.16 (s, OCH₂O-C), 155.00 (s, C_{Ar}-H), 190.26 (s, C=O); | 402 [M⁺, 26], 345 [M⁺, - O(CH₂)₃CH₃, 35]; |
| **3a"** | 0.86-0.94 (m,3H, OCH₂(CH₂)₁₄CH₃), 1.19-1.29 (m 28H, OCH₂(CH₂)₁₄CH₃), 3.48 (s 3H, OCH₃), 3.46 (t, 2H, OCH₂(CH₂)₁₄CH₃), 3.79 (s, 6H, OCH₃), 3.80 (s, 3H, OCH₃), 6.93 (s, 1H, OCH₂O), 5.24 (s, 1H, OCH₂O), 6.04 (s, 1H, OCHO), 3.01 (s, 1H, CHOC₁₆H₃₃), 7.01 (s, 2H, Ar-H), 7.24 (s, 1H, Ar-H), 7.51 (s, 1H, Ar-H), 10.16 (s, 1H, CHO); | 14.05 (s, CH₂), 22.62 (s, CH₂), 26.26 (s, CH₂), 33.01 (s, CH₂), 56.03 (s, OCH₃), 60.73 (s, OCH₃), 69.59 (s, OCH₂(CH₂)₁₄CH₃), 79.45 (s, CHO(CH₂)₃CH₃), 102.04 (s, OCH₂O), 103.92 (s, C_{Ar}-H), 108.21 (s, C_{Ar}-H), 109.58 (s, C_{Ar}), 128.45 (s, C_{Ar}), 137.08 (s, C-CHOC₁₆H₃₃), 137.26 (s, C_{Ar}), 141.81 (s, C_{Ar}), 147.48 (s, C-OCH₂O), 152.34 (s, OCH₂O-C), 153.20 (s, C_{Ar}-H), 189.91 (s, C=O); | 571 [M⁺¹, 37], 330 [M⁺¹,-O(CH₂)₁₅CH₃, 100]; |
| **3b** | 3.09 (s, 3H, OCH₃), 3.28 (s, 3H, OCH₃), 5.12 (s, 1H, OCH₂O), 5.16 (s, 1H, OCH₂O), 5.93(s, 1H, CHOMe), 7.30 (s, 1H, Ar-H), 7.35-7.39 (m, 1H, Ar-H), 7.66 (s, 1H, Ar-H), 7.82 (m, 1H, Ar-H), 10.02 (s, 1H, CHO); | | 300 [M⁺, 100] |
| **3d** | 3.06 (s, 3H, OCH₃), 3.28 (s, 6H, OCH₃), 3.74 (s, 3H, OCH₃), 5.87(s, 1H, CHOMe), 6.69 (s, 2H, Ar-H), 6.95-6.99 (m, 2H, Ar-H), 7.28-7.32 (m, 1H, Ar-H), 7.95-8.00 (m, 1H, Ar-H), 10.64 (s, 1H, CHO); | 55.72 (s, CH₃), 56.55 (s, CH₃), 62.71 (s, CH₃), 80.82 (s, CHOMe), 105.29 (s, C_{Ar}-H), 123.40 (s, C_{Ar}H), 124.15 (s, C_{Ar}H), 125.69 (s, C_{Ar}H), 126.73 (s, (s, C_{Ar}), 136.36 (s, C_{Ar}), 139.34 (s, C_{Ar}), 139.89 (s, C_{Ar}), 141.74 (s, C_{Ar}), 143.19 (s, C_{Ar}), 145.06 (s, C_{Ar}-H), 181.81 (s, C=O); | 373 [M⁺¹, 30], 341 [M⁺¹, - MeOH, 100] |

### Example VIII. General procedure for synthesis of compounds with the formula 4 (workup and purification improvements).

A solution of protected orto-bromoaldehyde **5** (1 mmol) in dry THF was cooled down to -78°C, n-Buli (1.1 mmol) was added and the resulting mixture was stirred at this temperature for 15 minutes. Then, aldehyde **7** (1.2 mmol) was added and the mixture was slowly heated up to the room temperature, with stirring. NH₄Cl aqueous solution was added, and then the mixture was saturated with solid chloride, extracted with ethyl acetate and washed with water until neutral pH was obtained. After drying over MgSO₄ and evaporation of solvents, the product was purified by simple crystallization. Compounds of formulas **4a-h** were obtained in high and quantitative yields.

Spectroscopic data of the obtained compounds are presented in

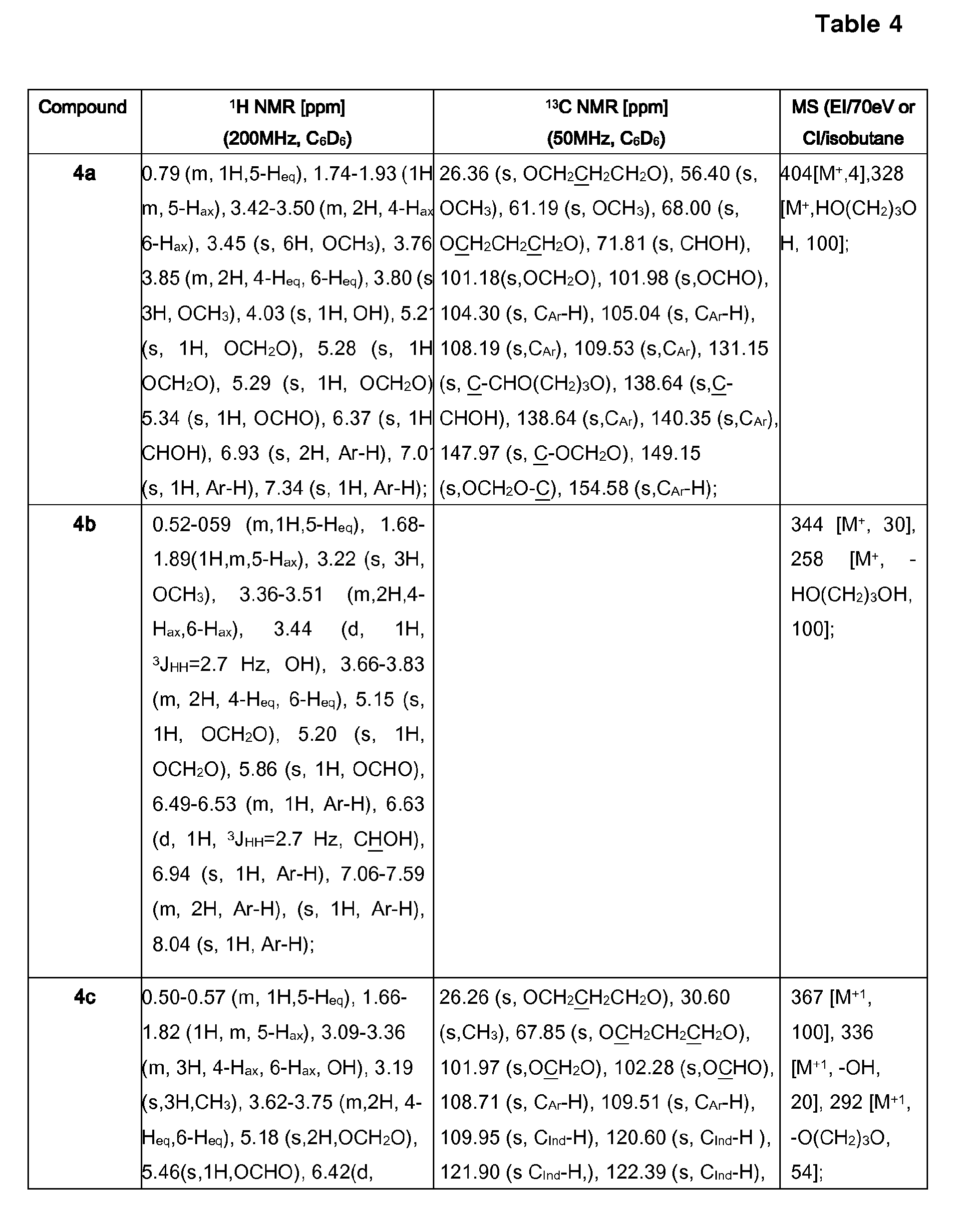

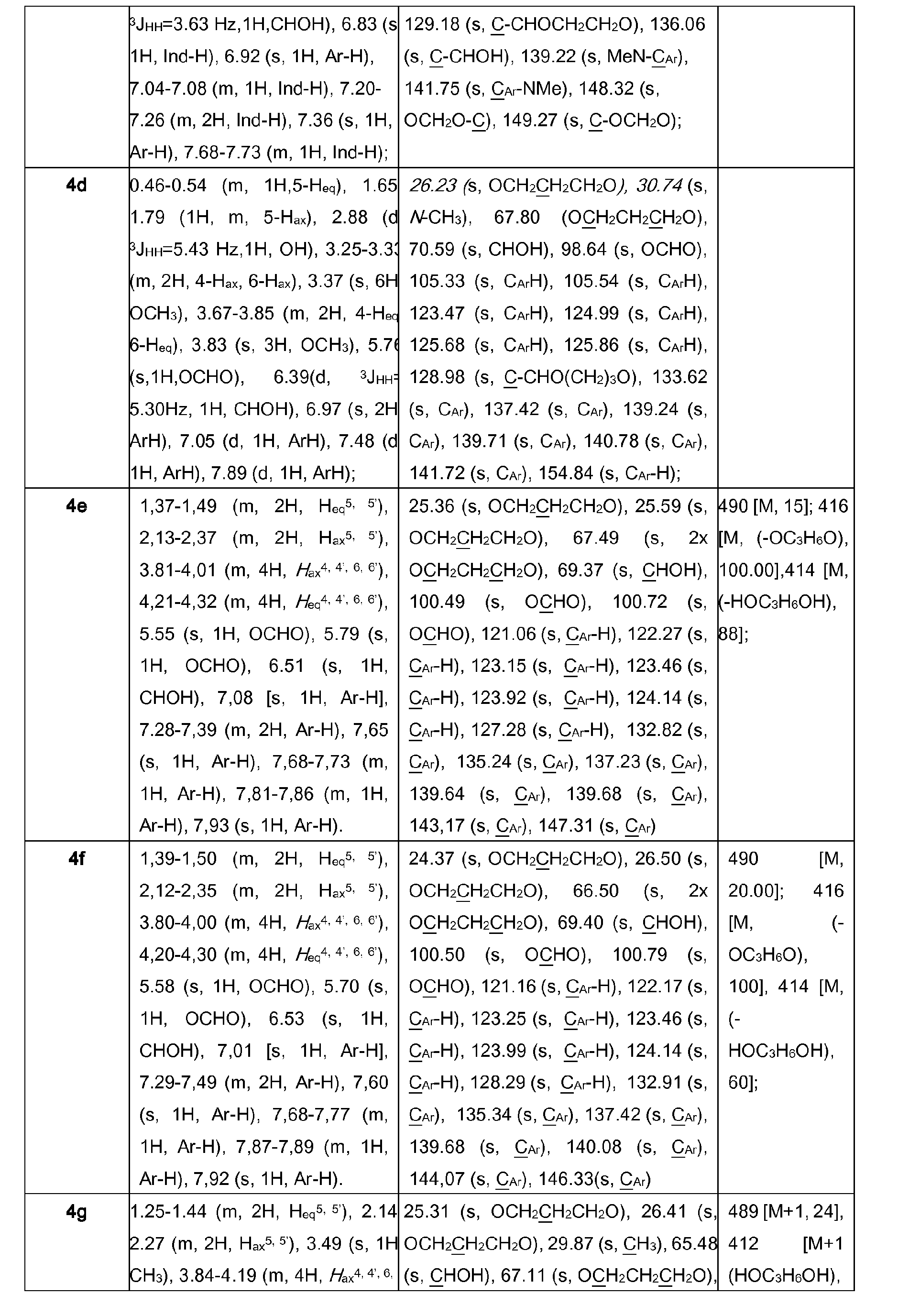

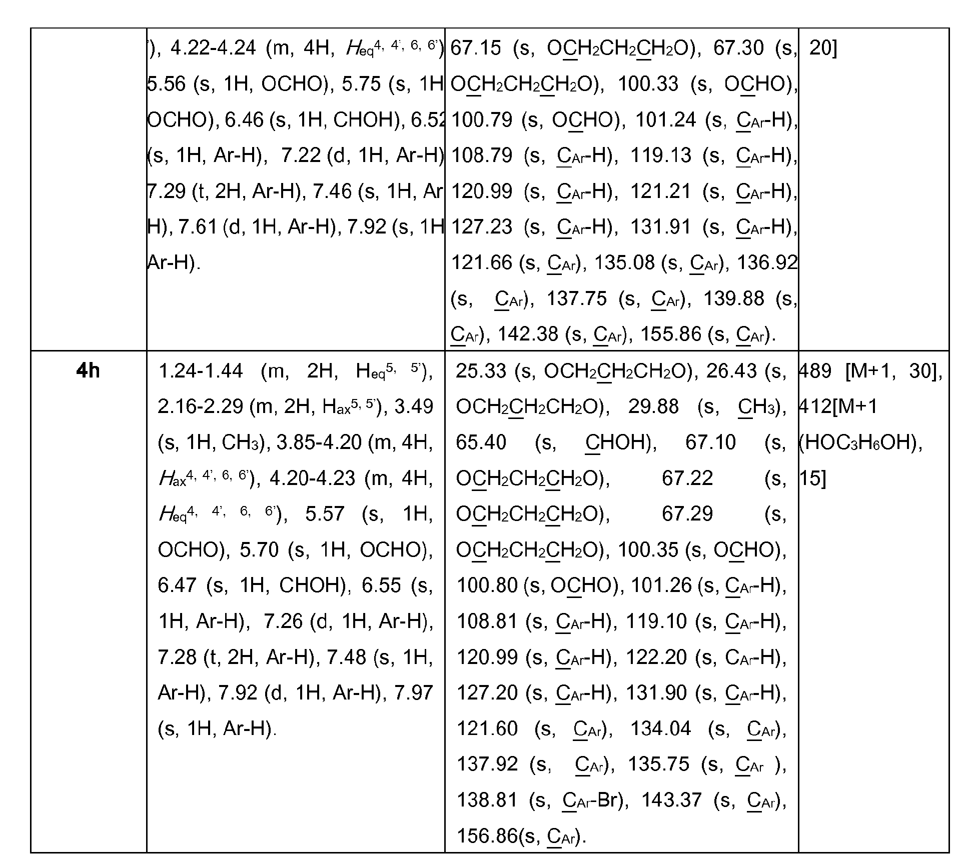

## Claims

1. A method of preparation of polycyclic, fused aromatic and heteroaromatic hydrocarbons with the formula 1, 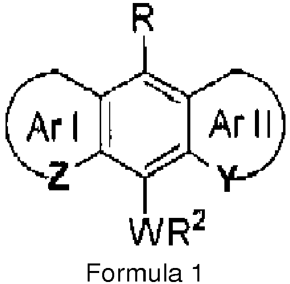 where:
W = oxygen or sulfur
Z and Y - are the same or different and stand for atoms of carbon or sulphur,
R - stands for hydrogen,
R2 - stands for alkyl, allyl,
ArI and ArII - are the same or different and stand for an aromatic ring, chosen from benzene, naphthalene, anthracene, phenantrene, or pyrene, or a five-membered heteroaromatic ring chosen from pyrrole, thiophene, furan, or six-membered heteroaromatic ring chosen pyridine, pyrimidine, pyran, thiopyran, or a mixed aromatic-heteroaromatic ring chosen from indole, benzothiophene, or benzofuran; mono- or multi-substituted with alkyl group containing 1 to 20 atoms of carbon, allyl group, benzyl group, aryl group, with dialkylamine or diarylamine group , halide groups F, Cl, Br, I, ketone, aldehyde, acyl, carboxyl, imine, alkoxy, aryloxy, nitrile, nitro, and/or heteroaryl groups, **characterized by** that, compounds with the formula 2, or with the formula 3, where Z, Y, R, R², ArI, ArII are defined as above and
R---R stands for a cyclic acetal derivative 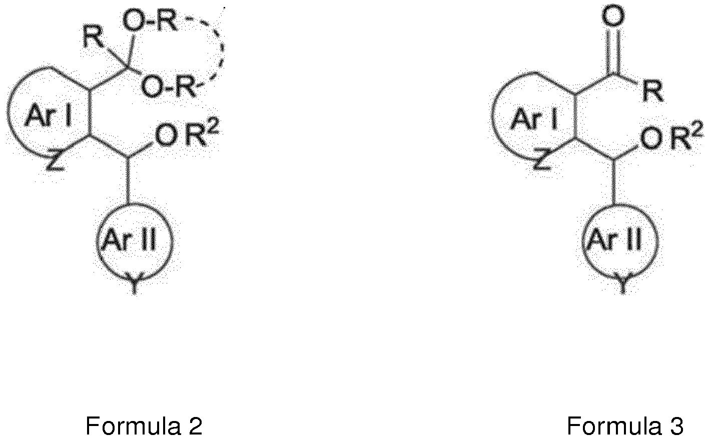 are transformed by an intramolecular cyclization reaction to compounds of formula 1 in a reaction time of up to 3 hrs whereby the reaction of transformation being carried out with the use of ultrasounds at room temperature or up to 60°C, in acidic environment, in a single reaction vessel, without separation of intermediate products.

2. The method according to the claim 1, **characterized in that**, it uses inorganic acids in any concentrations, gaseous HCl, organic acids, strong acidic ion-exchange resins, Lewis acids, as acidic reagents.

3. The method according to the claim 2, wherein the inorganic acids are selected from the group consisting of HCl, HBr, HI, HF, H₂SO₄, H₃PO₄.

4. The method according to the claim 2, wherein organic acids are selected from the group consisting of acetic, trifluoroacetic, methanesulfonic, trifluoromethanesulfonic, p-toluenesulfonic.

5. The method according to the claim 2, wherein strong acidic ion-exchange resins are selected from the group consisting of Amberlite® IR 120, Amberlyst® 15.

6. The method according to the claim 2, wherein Lewis' acids are selected from the group consisting of FeCl₃, ZnCl₂, SnCl₂·H₂O, SnCl₄.

7. The method according to the claim 1, **characterized in that**, the reaction is carried out using a combination of two or more acidic reagents of the same or different types or occurring in the same or different phases.

8. The method according to the claim 1, **characterized in that**, it uses water, mixtures of water-miscible organic solvents in any ratios, or organic solvents alone, as a solvent.

9. The method according to the claim 8, **characterized in that**, it uses water, mixtures of water-miscible organic solvents in any ratios, or organic solvents alone, selected from the group consisting of alcohols, ketones, ethers, alcohol ethers, aprotic solvents.

10. The method according to the claim 9, wherein alcohols are selected from the group consisting of methanol, ethanol, n-propanol, iso-propanol, n-butanol, ethylene glycol, 1,3-propandiol, glycerol, 3-oxapentano-1,5-diol.

11. The method according to the claim 9, wherein ketones are selected from the group consisting of acetone, ethyl methyl ketone.

12. The method according to the claim 9, wherein ethers are selected from the group consisting of 1,2-dimethoxyethane (DME), tetrahydrofuran (THF), 1,4-dioxane, dimethyl ether of ethylene glycol.

13. The method according to the claim 9, wherein alcohol ether is monomethyl ether of ethylene glycol.

14. The method according to the claim 9, wherein aprotic solvents are selected from the group consisting of, dimethylformamide (DMF), formamide, dimethyl sulfoxide (DMSO), hexamethylphosphoramide (HMPA), sulfolane, acetonitrile.

15. The method according to the claim 1, **characterized in that**, the compounds with the formula 2 are obtained from compounds with formula 4 by protection of the hydroxyl group in the compounds 4 within 12 hours, wherein the variables are as defined in claim 1.

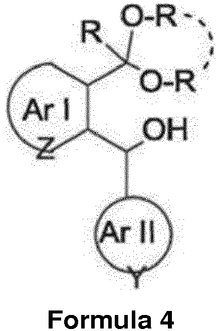

16. The method according to the claim 1, **characterized in that**, the compounds with the formula 3 are obtained from reaction of deacetalization of the compounds 2 in boiling toluene, in the presence of 1N HCl, within 12 hours, and the post-reaction mixture is salted out with saturated sodium chloride solution.

17. The method according to the claim 1, **characterized in that**, the compounds with the formula 4 are obtained in the way involving reactions of protected orto-bromo aldehydes 5, wherein Hal is bromo, and K and L are oxygen with aromatic or heteroaromatic aldehydes 7, wherein the variables are as defined in claim 1, in presence of n-BuLi, and salting out post-reaction mixtures with saturated sodium chloride solution.

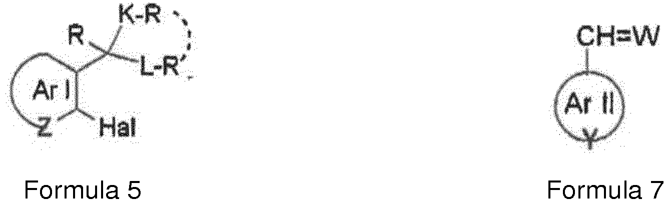

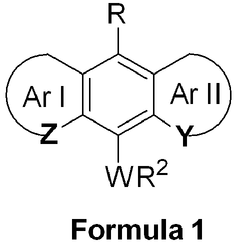

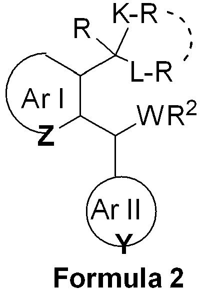

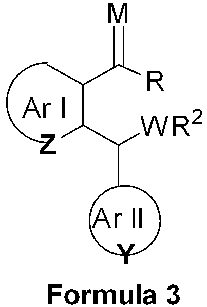

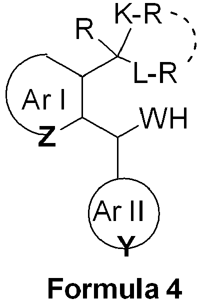

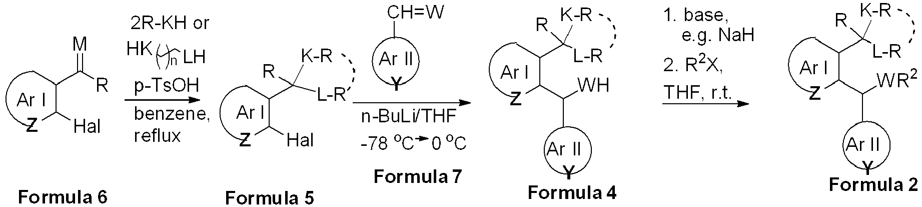

## Patentansprüche

1. Das Verfahren zur Herstellung von polyzyklischen, verbundenen aromatischen und heteroaromatischen Kohlenwasserstoffen mit der Formel 1, 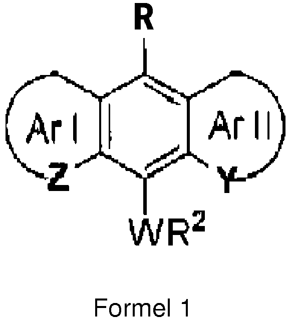 wo:
W = Sauerstoff oder Schwefel
Z und Y - sind gleich oder verschieden und stehen für Atome von Kohlenstoff oder Schwefel,
R - steht für Wasserstoff,
R2 - steht für Alkyl, Allyl,
Arl und ArII - sind gleich oder verschieden und stehen für einen aromatischen Ring, ausgewählt aus Benzyl, Naphthalin, Anthracen, Phenantren oder Pyren, oder einen fünfgliedrigen heteroaromatischen Ring, ausgewählt aus Pyrrol, Thiophen, Furan oder einem sechsgliedrigen heteroaromatischen Ring, ausgewählt aus Pyridin, Pyrimidin, Pyran, Thiopyran oder einem gemischten aromatischheteroaromatischen Ring, ausgewählt aus Indol, Benzothiophen oder Benzofuran; ein- oder mehrfach substituiert mit einer Alkylgruppe mit 1 bis 20 Kohlenstoffatomen, einer Allylgruppe, einer Benzylgruppe, einer Arylgruppe, einer Dialkylamin- oder Diarylamingruppe, Halogenidgruppen F, Cl, Br, I, Keton, Aldehyd, Acyl, Carboxyl, Imin, Alkoxy, Aryloxy, Nitril, Nitro und/oder Heteroarylgruppen, **dadurch gekennzeichnet, dass** die Verbindungen der Formel 2 oder der Formel 3, worin Z, Y, R, R2, Arl, Arll wie vorstehend definiert sind,
und
R--- R für ein zyklisches Acetalderivat steht 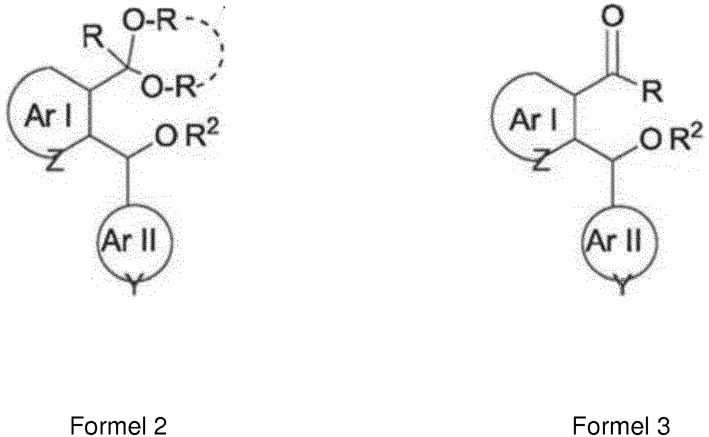 durch eine intramolekulare Zyklisierungsreaktion auf die Verbindungen der Formel 1 in einer Reaktionszeit von bis zu 3 h umgewandelt werden, wobei die Umwandlungsreaktion unter Verwendung von Ultraschall bei Raumtemperatur oder bis zu 60°C in saurer Umgebung in einem einzigen Reaktionsgefäß ohne Trennung von Zwischenprodukten durchgeführt wird.

2. Das Verfahren nach Anspruch 1, **dadurch gekennzeichnet, dass** es anorganische Säuren in beliebigen Konzentrationen, gasförmiges HCl, organische Säuren, stark saure lonenaustauscherharze, Lewis-Säuren als saure Reagenzien verwendet.

3. Das Verfahren nach Anspruch 2, wobei die anorganischen Säuren aus einer Gruppe, bestehend aus HCl, HBr, HI, HF, H₂SO₄, H₃PO₄, ausgewählt sind.

4. Das Verfahren nach Anspruch 2, wobei die organischen Säuren aus einer Gruppe, bestehend aus Essig, Trifluoressig, Methansulfon, Trifluormethansulfon, p-Toluolsulfon, ausgewählt sind.

5. Das Verfahren nach Anspruch 2, wobei stark saure lonenaustauscherharze aus einer Gruppe, bestehend aus Amberlite® IR 120, Amberlyst® 15, ausgewählt sind.

6. Das Verfahren nach Anspruch 2, wobei die Lewis-Säuren aus einer Gruppe, bestehend aus FeCl₃, ZnCl₂, SnCl₂ H₂O, SnCl₄, ausgewählt sind.

7. Das Verfahren nach Anspruch 1, **dadurch gekennzeichnet, dass** die Reaktion unter Verwendung einer Kombination von zwei oder mehreren sauren Reagenzien gleicher oder unterschiedlicher Art oder in gleichen oder unterschiedlichen Phasen durchgeführt wird.

8. Das Verfahren nach Anspruch 1, **dadurch gekennzeichnet, dass** es Wasser, Mischungen von mit Wasser mischbaren organischen Lösungsmitteln in beliebigen Verhältnissen oder organische Lösungsmittel allein als Lösungsmittel verwendet.

9. Das Verfahren nach Anspruch 8, **dadurch gekennzeichnet, dass** es Wasser, Mischungen von mit Wasser mischbaren organischen Lösungsmitteln in beliebigen Verhältnissen oder organische Lösungsmittel allein, ausgewählt aus einer Gruppe, bestehend aus Alkoholen, Ketonen, Ethern, Alkoholethern, aprotischen Lösungsmitteln, verwendet.

10. Das Verfahren nach Anspruch 9, wobei die Alkohole aus einer Gruppe, bestehend aus Methanol, Ethanol, n-Propanol, Iso-Propanol, n-Butanol, Ethylenglykol, 1,3-Propandiol, Glycerin, 3-Oxapentano-1,5-diol, ausgewählt sind.

11. Das Verfahren nach Anspruch 9, wobei Ketone aus einer Gruppe, bestehend aus Aceton, Ethylmethylketon, ausgewählt sind.

12. Das Verfahren nach Anspruch 9, wobei Ether aus einer Gruppe, bestehend aus 1,2-Dimethoxyethan (DME), Tetrahydrofuran (THF), 1,4-Dioxan, Dimethylether von Ethylenglykol, ausgewählt sind.

13. Das Verfahren nach Anspruch 9, wobei der Alkoholether Monomethylether von Ethylenglykol ist.

14. Das Verfahren nach Anspruch 9, wobei aprotische Lösungsmittel aus einer Gruppe, bestehend aus Dimethylformamid (DMF), Formamid, Dimethylsulfoxid (DMSO), Hexamethylphosphoramid (HMPA), Sulfolan, Acetonitril, ausgewählt sind.

15. Das Verfahren nach Anspruch 1, **dadurch gekennzeichnet, dass** die Verbindungen mit der Formel 2 aus Verbindungen mit der Formel 4 durch den Schutz der Hydroxylgruppe in den Verbindungen 4 innerhalb von 12 Stunden erhalten werden, wobei die Variablen wie in Anspruch 1 definiert sind.

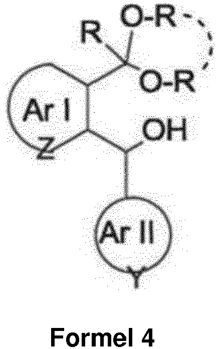

16. Das Verfahren nach Anspruch 1, **dadurch gekennzeichnet, dass** die Verbindungen mit der Formel 3 durch die Reaktion der Deacetalisierung der Verbindungen 2 in kochendem Toluol, in Gegenwart von 1N HCl, innerhalb von 12 Stunden erhalten werden und das Nachreaktionsgemisch mit gesättigter Natriumchloridlösung ausgesalzt wird.

17. Das Verfahren nach Anspruch 1, **dadurch gekennzeichnet, dass** die Verbindungen mit der Formel 4 in der Weise, die die Reaktionen von geschützten Orbromaldehyden 5 zur Folge hat, worin Hal Brom ist und K und L Sauerstoff mit aromatischen oder heteroaromatischen Aldehyden 7 sind, wobei die Variablen wie in Anspruch 1 definiert sind, in Gegenwart von n-BuLi und Salzen von Nachreaktionsgemischen mit gesättigter Natriumchloridlösung erhalten werden.

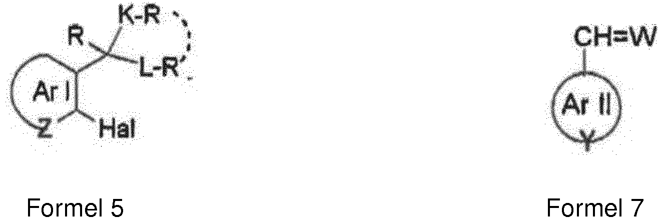

## Revendications

1. Une méthode de préparation d'hydrocarbures aromatiques et hétéroaromatiques polycycliques, fondus de formule 1, 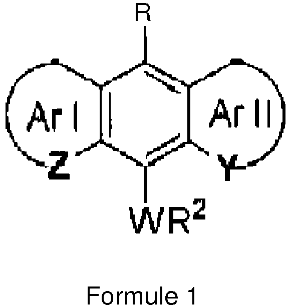 où :
W = oxygène ou soufre
Z et Y - sont identiques ou différents et représentent des atomes de carbone ou de soufre,
R - représente hydrogène,
R2 - représente alkyle, allyle,
Arl et Arll - sont identiques ou différents et représentent un cycle aromatique choisi parmi le benzène, le naphtalène, l'anthracène, le phénanthrène ou le pyrène, ou un cycle hétéroaromatique à cinq chaînons choisi parmi le pyrrole, le thiophène, le furanne ou un cycle hétéroaromatique à six chaînons choisi parmi la pyridine, la pyrimidine, le pyranne, le thiopyrane, ou un cycle mixte aromatique-hétéroaromatique choisi parmi l'indole, le benzothiophène ou le benzofurane ; mono- ou multi-substitués par un groupe alkyle contenant 1 à 20 atomes de carbone, groupe allyle, groupe benzyle, groupe aryle, avec groupe dialkylamine ou diarylamine, groupes halogénures F, Cl, Br, I, cétone, aldéhyde, acyle, carboxyle, imine, alcoxy, aryloxy, nitrile, nitro et / ou groupes hétéroaryle, **caractérisés en ce que** les composés de formule 2 ou de formule 3, où Z, Y, R, R2, Arl, Arll sont définis comme ci-dessus
et
R --- R représente un dérivé de l'acétal cyclique 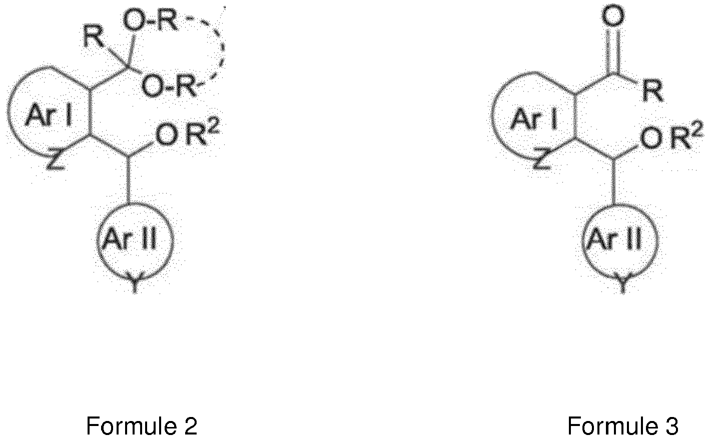 sont transformées par une réaction de cyclisation intramoléculaire en composés de formule 1 en un temps de réaction allant jusqu'à 3 heures, la réaction de transformation étant réalisée avec l'utilisation d'ultrasons à température ambiante ou jusqu'à 60°C, en milieu acide, dans un seul récipient de réaction, sans séparation des produits intermédiaires.

2. La méthode selon la revendication 1, **caractérisée en ce qu'**elle utilise des acides inorganiques à toutes concentrations, HCl gazeux, acides organiques, résines fortement acides échangeuses d'ions, acides de Lewis, en tant que réactifs acides.

3. La méthode selon la revendication 2, dans laquelle les acides inorganiques sont choisis dans le groupe comprenant HCl, HBr, HI, HF, H2S04, H3P04.

4. La méthode selon la revendication 2, dans laquelle les acides organiques sont choisis dans le groupe comprenant acétique, trifluoroacétique, méthanesulfonique, trifluorométhanesulfonique, p-toluènesulfonique.

5. La méthode selon la revendication 2, dans laquelle les résines fortement acides échangeuses d'ions sont choisies dans le groupe comprenant Amberlite® IR 120, Amberlyst® 15.

6. La méthode selon la revendication 2, dans laquelle les acides de Lewis sont choisis dans le groupe comprenant FeCl3, ZnCl2, SnCl2 H2O, SnCl4.

7. La méthode selon la revendication 1, **caractérisée en ce que** la réaction est effectuée en utilisant une combinaison de deux ou plusieurs réactifs acides de types identiques ou différents ou se produisant dans les phases identiques ou différentes.

8. La méthode selon la revendication 1, **caractérisée en ce qu'**elle utilise de l'eau, des mélanges de solvants organiques miscibles à l'eau dans des proportions quelconques, ou des solvants organiques seuls, en tant que solvant.

9. La méthode selon la revendication 8, **caractérisée en ce qu'**elle utilise de l'eau, des mélanges de solvants organiques miscibles à l'eau dans des proportions quelconques, ou des solvants organiques seuls, choisis dans le groupe comprenant les alcools, les cétones, les éthers, les éthers d'alcool, les solvants aprotiques.

10. La méthode selon la revendication 9, dans laquelle les alcools sont choisis dans le groupe comprenant le méthanol, l'éthanol, le n-propanol, l'isopropanol, le n-butanol, l'éthylène glycol, le 1,3-propanediol, le glycérol, le 3-oxapentano-1,5-diol.

11. La méthode selon la revendication 9, dans laquelle les cétones sont choisies dans le groupe comprenant l'acétone, l'éthyl méthyl cétone.

12. La méthode selon la revendication 9, dans laquelle les éthers sont choisis dans le groupe comprenant le 1,2-diméthoxyéthane (DME), le tétrahydrofurane (THF), le 1,4-dioxanne, le diméthyléther d'éthylène glycol.

13. La méthode selon la revendication 9, dans laquelle l'éther d'alcool est l'éther monométhylique d'éthylène glycol.

14. La méthode selon la revendication 9, dans laquelle les solvants aprotiques sont choisis dans le groupe comprenant le diméthylformamide (DMF), le formamide, le diméthylsulfoxyde (DMSO), l'hexaméthylphosphoramide (HMPA), le sulfolane, l'acétonitrile.

15. La méthode selon la revendication 1, **caractérisée en ce que** les composés de formule 2 sont obtenus à partir de composés de formule 4 par protection du groupe hydroxyle dans les composés 4 dans les 12 heures, les variables étant telles que définies dans la revendication 1.

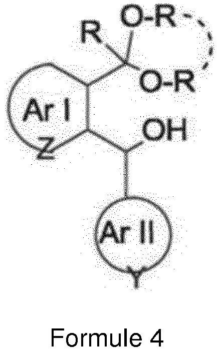

16. La méthode selon la revendication 1, **caractérisée en ce que** les composés de formule 3 sont obtenus par réaction de désacétalisation des composés 2 dans du toluène bouillant, en présence de HCl 1N, dans les 12 heures, et le mélange post-réactionnel est relargué avec une solution saturée de chlorure de sodium.

17. La méthode selon la revendication 1, **caractérisée en ce que** les composés de formule 4 sont obtenus de manière impliquant des réactions d'aldéhydes orto-bromo protégés 5, dans laquelle Hal représente brome, et K et L représentent oxygène avec des aldéhydes aromatiques ou hétéroaromatiques 7, dans laquelle les variables sont telles que définies dans la revendication 1, en présence de n-BuLi, et relargage de mélanges post-réactifs avec une solution saturée de chlorure de sodium.
